# EUROPEAN PATENT APPLICATION

(11) **EP 3 730 178 A1**
(43) Date of publication of application: **28.10.2020**
(21) Application number: 20171130.6
(22) Date of filing: 23.04.2020
(51) Int. Cl.: A61M 25/06

(54) **VASCULAR ACCESS SYSTEM**

(30) Priority: 24.04.2019 US 201916393627
(71) Applicant: DePuy Synthes Products, Inc., Raynham, MA 02767 (US)
(72) Inventor: SLAZAS, Robert, Raynham, MA 02767 (US)
(74) Representative: Small, Gary James

(57) **Abstract**

Vascular access system such as for radial artery access. An assembled integral dedicated unit including a guide catheter receivable within a lumen of an introducer sheath. The introducer sheath has a sidewall distal section, a sidewall proximal section and a sidewall transition section at an interface therebetween. An outer diameter of the sidewall distal section is less than an outer diameter of the sidewall proximal section. A method for using the vascular access system includes preparation for introduction through an access site, while radially supported by the guide catheter, sliding the introducer sheath over the guide catheter to the distal end to form the assembled integral dedicated unit. Back-loading in a proximal direction starting from a distal end of the assembled integral dedicated unit with the guidewire until the introducer sheath extends from an interior of the vessel to exteriorly of the access site.

## Description

### Field of the Invention

The present invention relates to a vascular access system. In particular, the present invention is directed to a vascular access system for access of any part of the vascular system (e.g., artery, vein, chamber, etc.). In a particular application, the present invention relates to a system and method for vascular access via the radial artery for treating the brain, heart or anywhere else in the body.

### Description of Related Art

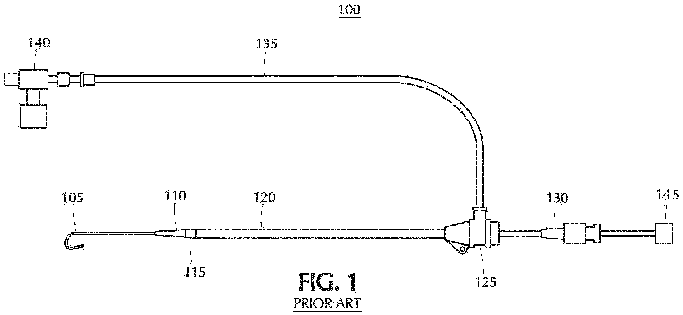

Numerous vascular (e.g., neurovascular and cardiovascular) treatments and diagnoses employ a therapeutic or diagnostic catheter, for example, a guiding catheter and a balloon catheter, advanced through a vessel of the body. Rather than introducing the therapeutic or diagnostic catheter directly into the body (i.e., bareback), an introducer sheath commonly serves as a portal to protect tissues and vessels at the access site from trauma and damage during advancement and manipulation of the catheter or other ancillary devices. The introducer sheath is commonly part of a conventional introducer assembly or kit. Referring to Figure 1, a conventional introducer system or kit 100 may include: a guidewire 105 having an atraumatic distal end or tip (e.g., a J-curved distal end or tip), a dilator (cone expander) 110, a radiopaque marker band 115, an introducer sheath or tube 120, a hub 125 (typically including a hemostasis ("hemo") valve) and a dilator hub 130. Proximally of the dilator hub 130, introducer system or kit 100 may further include a guide wire introducer 145, which is a relatively long and skinny cone that assists the guidewire 105 to either enter the dilator or penetrate the hemo valve in the introducer cap. Without the introducer 145, the flexible J-curved distal end or tip on the guidewire would prevent the wire from being introduced. A flexible side tube or port 135 in fluid communication with the hub 125 may also be employed. At a distal end of the side tube or port 135, opposite the hub 125, is a 3-way stopcock 140. Side tube or port 135 may be used for any number of functions such as administration of a fluid (e.g., flushing fluid, contrast fluid, and/or drug) and/or withdrawal of blood.

The introducer sheath 120 comprises a tube segment of biocompatible material having a lumen defined longitudinally therethrough. An inner diameter of the lumen of the introducer sheath is sized according to the largest outer diameter catheter to be accommodated therein. The dilator 110 disposed at the distal end of the introducer system or kit 100 is received within the lumen of the introducer sheath 120 and enlarges the puncture opening of the skin at the access site or point on the body sufficient to permit access therethrough of the introducer sheath 120. Dilator 110 is a relatively short, stiff, thick-walled section of catheter having a tapered distal end (typically forming a cone) that enlarges a passage through the body tissues. The hemostasis valve 125 comprising part of the introducer assembly 100 allows interchangeability of ancillary devices with minimal, or no, loss of blood.

During the procedure the introducer sheath 120 is introduced into a puncture site of the body with the dilator 110 positioned within the lumen thereof proximate at its distal end. The tapered design of the dilator 110 (with its distal end having the smallest diameter and increasing in diameter to its opposite proximal end having the largest diameter) enlarges or expands the passage through the body tissue surrounding the puncture site sufficient to accommodate the outer diameter of the introducer sheath 120. Once the introducer sheath 120 has been properly positioned proximate the access site of the body, the dilator 110 may be withdrawn proximally through the lumen of the introducer sheath 120. A catheter and/or other ancillary devices may thereafter be advanced therethrough the lumen of the introducer sheath 120 and into the vessel until reaching the target site in the body without causing damage to the surrounding body tissues.

The introducer sheath is typically color-coded following accepted industry standards, wherein different colors denote a maximum outer diameter of an ancillary medical device (for example, a catheter) accepted or accommodated in the lumen of the introducer sheath. Red color introducer sheath is able to accommodate or accept a 4F guide catheter; gray color introducer sheath is able to accommodate or accepts a 5F guide catheter; green color introducer sheath is able to accommodate or accept a 6F guide catheter; orange color introducer sheath is able to accommodate or accept a 7F guide catheter; blue color introducer sheath is able to accommodate or accepts a 8F guide catheter; black color introducer sheath is able to accommodate or accept a 9F guide catheter; purple color introducer sheath is able to accommodate or accept a 10F guide catheter; yellow color introducer sheath is able to accommodate or accept a 11F guide catheter.

By way of illustrative example, Table 1 below is illustrative of several conventional size guide catheters and corresponding conventional introducer sheath dimensions.

**TABLE 1:**

| | **6F Guide Catheter** | **7F Guide Catheter** | **8F Guide Catheter** |
|---|---|---|---|
| **Guide Catheter-Inner Diameter** | 0.070" | 0.078" | 0.088" |
| **Guide Catheter-Outer Diameter** | 0.082" | 0.094" | 0.105" |
| **Femoral Artery Introducer Sheath - Outer Diameter** | 0.109" | 0.122" | 0.133" |
| **Clearance between the outer diameter of the Introducer Sheath and the outer diameter of the Guide Catheter** | 0.027" | 0.028" | 0.028" |

Heretofore, the femoral artery located in the groin was the preferred access site (i.e., puncture point) at which the conventional introducer assembly 100 in Figure 1 is introduced into the body. Its relatively large inner diameter (typically, approximately 9.8 mm in males and approximately 8.2 mm in females) makes the femoral artery a preferred site for the introduction of the introducer sheath. A 6F catheter uses an introducer sheath having an outer diameter of 0.109"/2.7686 mm; a 7F catheter uses an introducer sheath having an outer diameter of 0.122"/3.0988 mm; or an 8F catheter uses an introducer sheath having an outer diameter of 0.133"/3.3782 mm. The inner diameter of the femoral artery typically increases with age and varies among individuals based on such factors as sex, age, and body size. Despite such clear advantage, several issues arise in using the femoral artery as an access site into the body during a surgical procedure that outweigh the benefit of size. The femoral artery poses numerous substantial deleterious health risks. Leakage or bleeding during and after the procedure is not uncommon when the femoral artery is used as an access point into the body.

The present invention is directed to a vascular access system and method with an improved introducer sheath design that when combined with a conventional size guide catheter to form an assembled unit introduced simultaneously through the access site overcomes the aforementioned problems.

### Summary of the Invention

An aspect of the present invention is directed to a vascular access system and method, preferably a radial artery access system and method, with an improved introducer sheath design that when combined with a conventional size guide catheter to form an assembled unit introduced simultaneously through the access site overcomes the aforementioned problems associated with conventional introducer sheaths.

Another aspect of the present invention relates to a vascular access system including an assembled integral dedicated unit. The assembled integral dedicated unit includes: an introducer sheath having a proximal end, an opposite distal end and a lumen defined axially therethrough from the proximal end to the distal end. The lumen of the introducer sheath having an inner diameter uniform along an entire length of the introducer sheath from the proximal end to the opposite distal end. Wherein, the introducer sheath has a sidewall distal section, a sidewall proximal section and a sidewall transition section at an interface therebetween. An outer diameter of the sidewall distal section is less than an outer diameter of the sidewall proximal section. The assembled integral dedicated unit further including a guide catheter having a lumen defined axially therethrough from a proximal end to an opposite distal end, wherein the guide catheter is receivable within the lumen of the introducer sheath.

Still another aspect of the present invention is directed to a method for using the vascular access system as described in the preceding paragraph. An access site is penetrated using a needle. An atraumatic guidewire is distally advanced through the needle to a target site a predetermined distance beyond the access site. Maintaining in position the guidewire, the needle is withdrawn from the access site. While the guidewire is maintained in position, the dilator, guide catheter and the introducer sheath are assembled in preparation for introduction through the access site. Specifically, such assembling step involves (i) inserting the cone at the distal end of the dilator into and advancing distally through the lumen of the guide catheter until the cone of the dilator fully emerges from the distal end of the guide catheter; and (ii) while radially supported by the guide catheter, sliding the introducer sheath over the guide catheter to the distal end of the guide catheter adjacent to the cone of the dilator. Then the assembly including the dilator, guide catheter and introducer sheath is back-loaded in a proximal direction starting from a distal end of the assembly with the guidewire. The assembly together as a single unit until the dilator cone is advanced interiorly beyond the access site so that the introducer sheath extends from interiorly of the access site to exteriorly of the access site. Withdrawing the dilator from the access site, the assembled guide catheter and introducer sheath are maintained in position. Lastly, the guide catheter is advanced to the target site, through the axial lumen defined in the introducer sheath, without damaging the access site shielded by the introducer sheath.

Yet another aspect of the present invention is directed to a method for using a vascular access system, as described above. At a desired location, an access site it cut down using a surgical cutting tool (e.g., scalpel). An atraumatic guidewire is advanced distally through the access site to a target site a predetermined distance beyond the access site. While the guidewire is maintained in position, the guide catheter and the introducer sheath are assembled in preparation for introduction through the access site. Specifically, such assembling involves, while radially supported by the guide catheter, sliding the introducer sheath over the guide catheter to the distal end of the guide catheter. The assembly including the guide catheter and introducer sheath are back-loaded in a proximal direction starting from a distal end of the assembly with the guidewire. Then, the assembly together as a single unit is advanced until interiorly beyond the access site so that the introducer sheath extends from interiorly of the access site to exteriorly of the access site. Lastly, the guide catheter is advanced to the target site, through the axial lumen defined in the introducer sheath, without damaging the access site shielded by the introducer sheath.

### Brief Description of the Drawing

The foregoing and other features of the present invention will be more readily apparent from the following detailed description and drawings illustrative of the invention wherein like reference numbers refer to similar elements throughout the several views and in which:
Figure 1 illustrates a Prior Art femoral artery access system including an introducer sheath and guide catheter;
Figure 2A depicts the radial artery in the body of a woman;
Figure 2B is an enlarged view of the radial artery running down the left arm of the woman in Figure 2A;
Figure 3A is a longitudinal cross-sectional view of the introducer sheath with a guide catheter received therein to form the assembled integral dedicated unit comprising part of the vascular access system in accordance with the present invention, wherein a tapered step sidewall transition section is provided at the interface between the sidewall distal section and sidewall proximal section of the introducer sheath; and
Figure 3B is a longitudinal cross-sectional view of an alternative embodiment of the introducer sheath with a guide catheter received therein to form the assembled integral dedicated unit comprising part of the vascular access system in accordance with the present invention, wherein an annular shoulder sidewall transition section is provided at the interface between sidewall distal section and sidewall proximal section of the introducer sheath; and
Figure 4A is an enlarged view of the dashed area IV-A of Figure 3A, wherein the sidewall transition section of the introducer sheath is a tapered step transition;
Figure 4B is an enlarged view of the dashed area IV-B of Figure 3B, wherein the sidewall transition section of the introducer sheath is an annular shoulder transition;
Figure 5A depicts a cross-sectional view of the needle puncturing the skin at an access point and the guidewire being advanced therethrough; and
Figure 5B depicts a partial cross-sectional view of the assembled dilator, guide catheter and introducer sheath comprising part of the radial artery access system in accordance with the present invention back-loaded over the guidewire.

### Detailed Description of the Invention

The terms "distal" or "proximal" are used in the following description with respect to a position or direction relative to the treating physician or medical interventionalist. "Distal" or "distally" are a position distant from or in a direction away from the physician or interventionalist. "Proximal" or "proximally" or "proximate" are a position near or in a direction toward the physician or medical interventionist. The terms "occlusion", "clot" or "blockage" are used interchangeably.

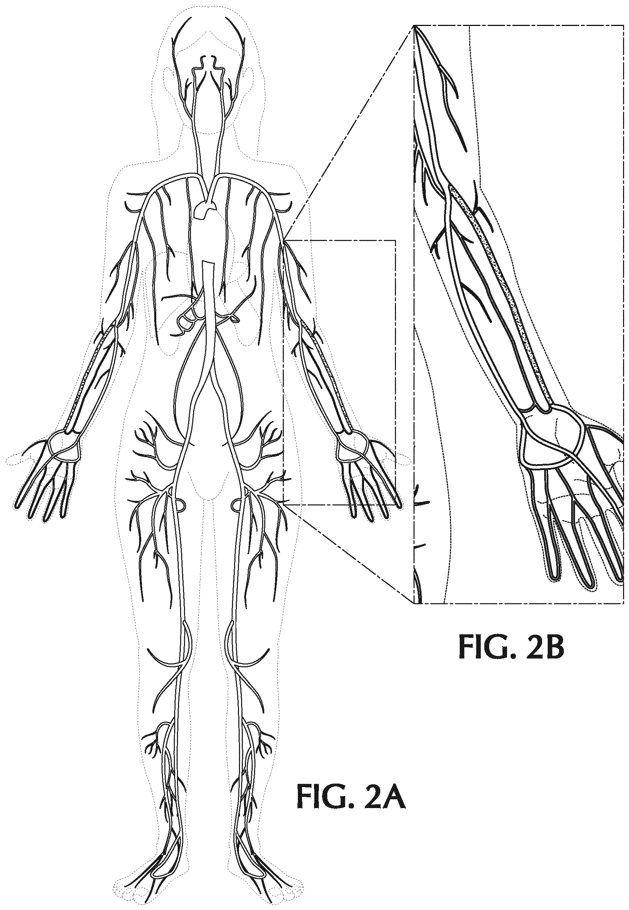

The present invention minimizes the occurrence of health complications by providing alternative access points in the vascular system (e.g., via the radial artery) for surgical procedures to the brain, heart and anywhere else in the body while still accommodating conventional size guide catheters (e.g., 6F, 7F, 8F size catheters). By way of illustrative example only, the present invention is shown and described as access via the radial artery. However, the present invention is suitable to provide vascular access anywhere in the vascular system. Referring to Figures 2A & 2B, the radial artery is close to the surface and runs along the underside of the forearm when the palm of the hand is pointing upwards. In comparison to that of the femoral artery, potential risk of leakage or bleeding when using the radial artery as the access point is substantially less. Moreover, since the radial artery is shallow (i.e., located closer to the skin) in comparison to that of the femoral artery, any leakage or bleeding, should it occur, is more easily detectible and readily addressed. Use of the radial artery as the access point has the added benefit of allowing direct entry to the brain, i.e., without having to pass first through the aorta in many anatomical variations, when accessing the same side of the brain as the arm selected. A still further advantage to the radial artery being used as an access point is that in the unlikely event that blood flow is cut off completely in the artery used as the access site due to complications during the procedure, other parallel, "collateral" arteries continue to supply oxygenated blood from the lungs to the hand and arm. Thus, blood flow to the hand and arm may be reduced, but won't cease altogether, having less deleterious health effects on the patient.

Despite such benefits, the substantially smaller size of the radial artery relative to that of the femoral artery poses a significant restriction on the size of the catheter to be accommodated or accepted therein if a conventional introducer sheath is used. The size of the radial artery varies among individuals based on such factors as sex, diabetes and smoking. Studies have found that the mean inner diameter for men and women of the right radial artery on average is 2.44 ± 0.60 mm, which size may be unable to accommodate conventional introducer sheaths of 7F to 8F catheters.

The introducer sheath maintains a space in the body tissue at the point of entry (e.g., puncture site) sufficient in diameter to accommodate conventional guide catheters of varying diameters (e.g., 6F, 7F, 8F). This requires a relatively large differential clearance between the outer diameter of the introducer sheath and the outer diameter of the guide catheter. Referring to Table 1, the outer diameter of the conventional introducer sheath (6F introducer sheath having an outer diameter of 0.109"/2.7686 mm; 7F introducer sheath having an outer diameter of 0.122"/3.0988 mm; and 8F introducer sheath having an outer diameter of 0.133"/3.3782 mm) is too large to be received in the radial artery. Despite the relatively small diameter of the radial artery, based on body type some individuals may be able to accommodate or accept a 6F guide catheter and associated introducer sheath therein. But, in some people, 7F or 8F guide catheters and their associated conventional introducer sheath in Table 1 may be too large in size to be accommodated or accepted into the radial artery.

The smaller size radial artery in comparison to that of the femoral artery poses substantial challenges in design of the introducer sheath so as not to preclude use of conventional size guide catheters therewith. Irrespective of the reduced size of the access point, e.g., via the radial artery, the novel configuration of the present inventive vascular access system and method accommodate or accept a wide range of conventional size guide catheters (e.g., 6F, 7F, 8F).

A conventional introducer sheath is sequentially introduced into the access site before that of the guide catheter. The sidewall of the conventional introducer sheath must therefore be sufficiently thick to have the requisite radial strength to maintain its shape (e.g., self-supporting) when introduced into the access site following puncture and thereafter to permit the guide catheter to be delivered therethrough.

So that varying size conventional guide catheters (6F, 7F, 8F) may be utilized with the radial artery as an access point, the novel configuration of the present inventive introducer sheath focuses on reducing only a portion its outer diameter by thinning a sidewall distal section (relative to that of a conventional introducer sheath whose sidewall has a uniform thickness along its entire longitudinal length). However, as mentioned in the preceding paragraph, because the conventional introducer sheath is passed through the puncture or access site first, followed sequentially thereafter by the guide catheter, the thinned sidewall distal section or portion of the introducer sheath alone would be unable to structurally support itself. The present inventive radial artery access system overcomes this challenge by configuring the present inventive introducer sheath and guide catheter as an integral dedicated unit (i.e., assembled unit or assembled package) wherein the introducer sheath and guide catheter are introduced through the access site simultaneously (at the same time) as an integral dedicated unit. Because the present inventive introducer sheath and guide catheter are introduced through the access site as an integral dedicated unit (assembled unit or package), the required radial strength of the introducer sheath need not be provided by the introducer sheath itself (i.e., the introducer sheath need not be self-supporting). Rather, the requisite radial strength of the present inventive introducer sheath is supplied by the guide catheter comprising part of the integral dedicated unit.

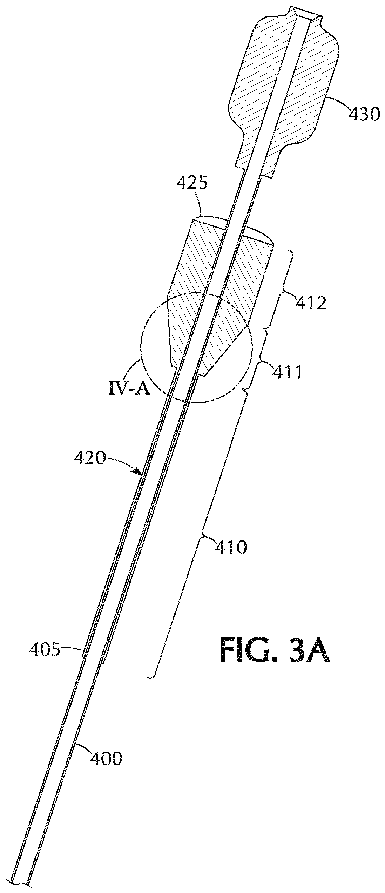

Figure 3A is a longitudinal cross-sectional view of the introducer sheath 420 of the present inventive vascular access system with a guide catheter 400 received therein. Introducer sheath 420 has a sidewall extending from a proximal end to its opposite distal end 405. The sidewall of the introducer sheath comprises a sidewall proximal section 412, followed by a sidewall transition section 411, followed distally thereafter by a sidewall distal section 410. In the example illustrated in Figure 3A the sidewall proximal section 412 together with the sidewall transition section 411 serve as an introducer sheath hub 425 (which preferably includes a hemo valve), with a guide catheter hub 430 arranged proximally thereof.

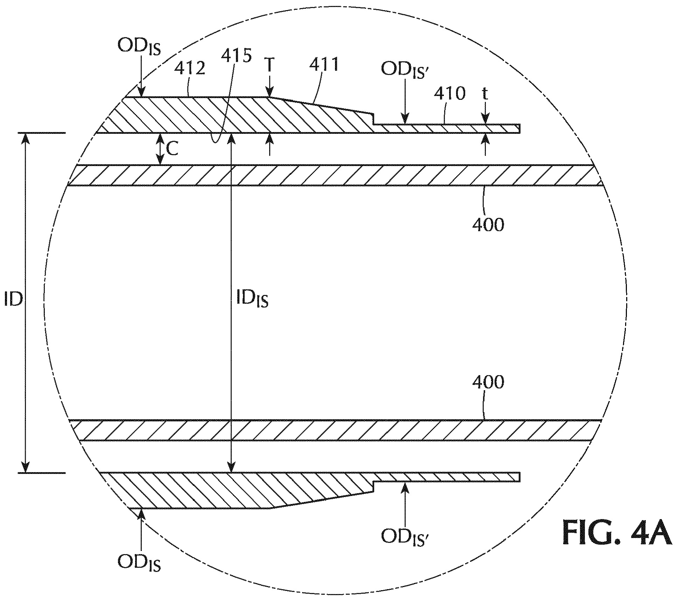

Figure 4A depicts an enlarged view of the circular dashed area IV-A of the assembled integral dedicated unit of Figure 3A. The inner diameter ID_{IS} of the introducer sheath 420 is uniform from its proximal end to its opposite distal end, while its outer diameter varies (i.e., non-uniform). Specifically, the sidewall of the introducer sheath 420 has a single sidewall transition section 411. In Figures 3A & 4A, the single sidewall transition section 411 is a tapered step (e.g., frustum cone) that defines an interface between the sidewall proximal section 412 and the sidewall distal section 410. Sidewall proximal section 412 (hereinafter referred to as a "thicker-sidewall proximal section") has a radial thickness "T", while sidewall distal section 410 (hereinafter referred to as a "thinner-sidewall distal section") has a reduced radial thickness "t", wherein the terms "thicker" and "thinner" are a comparison of the thickness "T" and "t" relative to one another. The thinner-sidewall distal section 410 is positioned distally relative to that of the thicker-proximal sidewall section 412. Thus, the overall outer diameter OD'_{IS} of the thinner-sidewall distal section 410 of the introducer sheath is less than that of the overall outer diameter OD_{IS} of the thicker-sidewall proximal section 412 of the introducer sheath. The reduced overall outer diameter OD'_{IS} of the thinner-sidewall distal section 410 of the present inventive introducer sheath 420 permits a larger size guide catheter 400 to be accepted into the radial artery than would be possible with a conventional introducer sheath having a sidewall of uniform thickness (e.g., 0.005") and uniform overall outer diameter along its entire longitudinal length. The thicker-sidewall proximal section 412 of the present inventive introducer sheath that remains external to the body preferably has a greater thickness "T" (relative to the thinner-sidewall distal section) for anchoring to the patient's skin using conventional anchoring techniques, and/or the optional inclusion of a hemostasis valve. The thinner-sidewall distal section 410 preferably has a thickness "t" between approximately 0.001" and approximately 0.002" defining an outer diameter OD'_{IS} between approximately 0.120" and approximately 0.118", respectively. Accordingly, the outer diameter OD'_{IS} of the distal sidewall section is preferably less than approximately 0.095" for a 6F guide catheter; preferably less than approximately 0.110" for a 7F guide catheter; or preferably less than approximately 0.120" for an 8F guide catheter. Preferably, the thicker-sidewall proximal section 412 has a thickness "T" equal to the uniform thickness of the conventional introducer sheath (e.g., approximately 0.005" - approximately 0.020"). To reduce the outer diameter of the distal section of the introducer sheath 420 still further the clearance or space "C" between the inner surface of a lumen 415 defined axially through the introducer sheath 420 and the outer surface of the guide catheter 400 may be minimized to the point of physical contact with one another. Thus, the thinning of the sidewall distal section of the introducer sheath alone or in combination with a reduction in the clearance between the introducer sheath and guide catheter provides a smaller overall total diameter of the introducer sheath able to accommodate a wide variety of manufactured guide catheters.

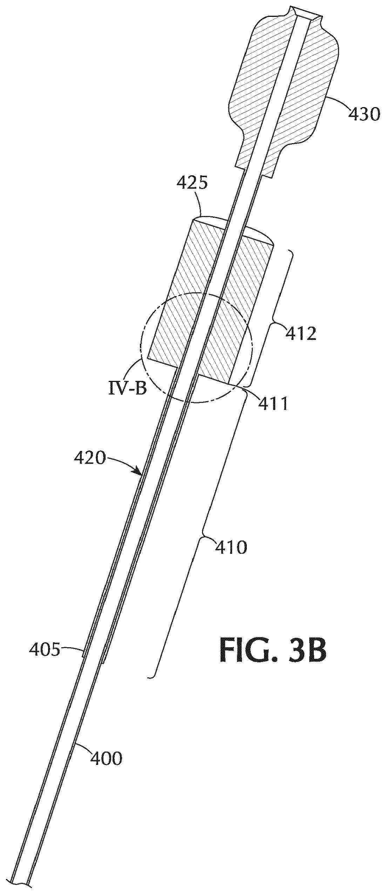

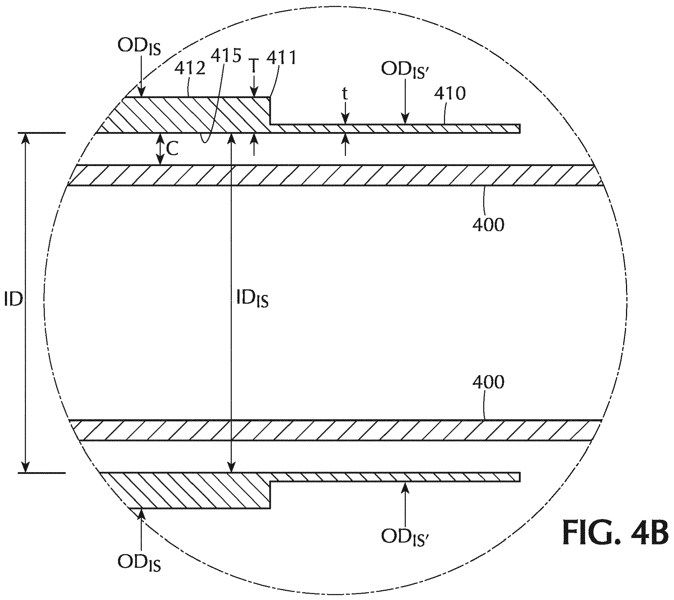

The sidewall thickness of conventional introducer sheaths is approximately 0.010" uniformly along its entire longitudinal or axial length. As previously noted, following puncture, such uniform sidewall thickness of the conventional introducer sheath provides the necessary structure to be self-supporting (without any physical structural support by any other component) to allow advancement sequentially and independently thereafter of the guide catheter therethrough. Because the guide catheter and present inventive introducer sheath are simultaneously introduced together through the puncture site as an assembled integral dedicated unit (i.e., single assembled package), the radial strength for the thinner-sidewall distal section 410 of the present inventive introducer sheath 420 is provided by the guide catheter 400 disposed within the lumen 415 of the introducer sheath comprising part of the integral dedicated unit, instead of by the introducer sheath itself. Accordingly, the thinner-sidewall distal section 410 of the present inventive introducer sheath 420 that is introduced into the body through the puncture or access site is no longer self-supporting (absent the structural support provided by the guide catheter disposed therein). In accordance with the present invention, the lumen 415 defined axially therethrough the introducer sheath 420 has an inner diameter ID_{IS} that is uniform along its entire longitudinal length from its proximal end to its opposite distal end. It is only the outer diameter of the present inventive introducer sheath 420 that is non-uniform, that is, the outer diameter OD'_{IS} of its thinner-sidewall distal section 410 is less than the outer diameter OD_{IS} of its thicker-sidewall proximal section 412. In the example illustrated in Figures 3A & 4A the sidewall transition section 411 represents a tapered step (e.g., frustum cone) at the interface between sidewall distal and sidewall proximal sections of the introducer sheath, wherein each of the sidewall distal and proximal sections has a uniform outer diameter. That is, the outer diameter OD'_{IS} of the thinner-sidewall distal section 410 is uniform axially along its entire length, while the outer diameter OD_{IS} of the thicker-sidewall proximal section 412 is also uniform axially along its entire length. In an alternative configuration depicted in Figures 3B & 4B, the sidewall transition section at the interface between the sidewall distal and proximal sections of the introducer sheath is an annular step or shoulder, instead of a tapered step (e.g., frustum cone) transition. The sidewall transition section may be any number of different configurations including, but not limited to, an annular step/shoulder, a cone (tapered to a point), or a combination thereof representing a tapered step (e.g., frustum cone tapered to a flat surface or step, rather than tapered to a point).

One or more of a lubricating fluid, a flushing fluid, or a drug may be delivered in the clearance space "C" between the inner wall of the lumen 415 of the present inventive introducer sheath 420 and the outer surface of the guide catheter 400, by way of an integral port at the proximal section of the introducer sheath. The present inventive introducer sheath is preferably made of a lubricious, biocompatible material such as fluoropolymer, for example, PTFE (e.g., Teflon®).

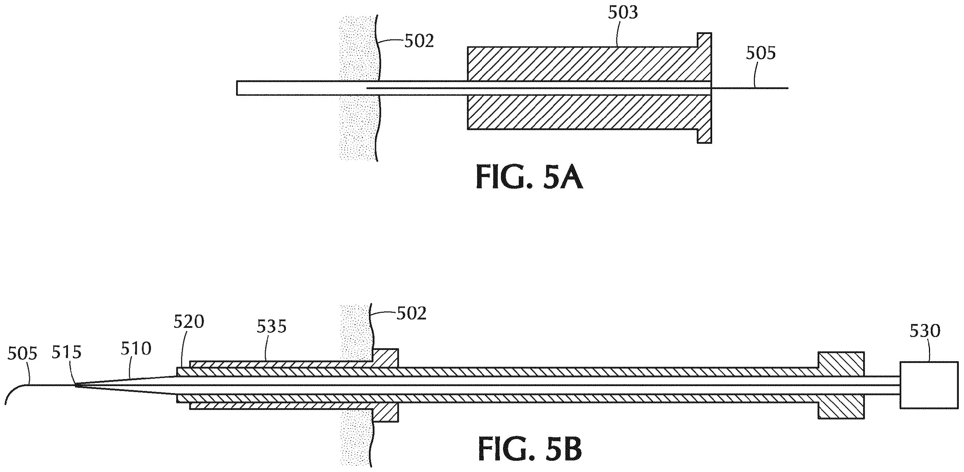

During the medical procedure, a needle 503 is used by the interventionalist to puncture the skin and penetrate the radial artery at the desired access location or site, typically near the wrist. An atraumatic guidewire 505 (preferably with a flexible/ J-shaped tip) is advanced distally through the needle to a targeted position within the radial artery a relatively short distance past the access point, toward the heart, as shown in Figure 5A. Guidewire 505 is maintained in position, while the needle is withdrawn proximally from the body. While the guidewire 505 is maintained in place, the dilator 510, guide catheter 520 and introducer sheath 535 are assembled in preparation for introduction as a unit or package through the access site. Specifically, the cone at the distal end of the dilator 510 (opposite the hub 530 at its proximal end) is inserted into and advanced distally through the entire length of the lumen of the guide catheter 520 until the tapered cone of the dilator fully emerges from the distal end of the guide catheter. Next, the present inventive introducer sheath 535 having an annular step or shoulder sidewall transition section in the example depicted is positioned so that the thinner-sidewall distal section, while radially supported by the guide catheter 520, is slid over the guide catheter (i.e., the guide catheter is advanced through the lumen of the introducer sheath 535) to the distal end of the guide catheter adjacent to the cone of the dilator 510. Dilator 510 has a relatively small lumen defined axially therethrough that is sized to receive the guidewire 505. Advantageously, dilator 510 increases the overall stiffness of the assembled unit which is beneficial during insertion of the assembled unit through the access site. The assembly comprising the dilator, guide catheter and introducer sheath is then "back-loaded" (e.g., loaded in a proximal direction starting from its distal end) with the guidewire 505, as shown in Figure 5B. In particular, the proximal end of the guidewire 505 is received within a distal opening 515 in the dilator 510 (e.g., at the tip of the cone) and advanced through the relatively small lumen defined axially therein until reaching the access site where the guidewire 505 enters the skin 502. As the assembly comprising the dilator, guide catheter and introducer sheath is further advanced in a distal direction the tapered cone of the dilator widens the access site through the skin and into the radial artery. The assembly is advanced until the dilator cone is past the access site and entirely within the radial artery while the present inventive introducer sheath extends from the interior of the access site to the exterior of the access site (i.e., the outside surface of the skin). During the treatment procedure itself, flexibility is of primary importance while navigating the tortuous path of the vessel by the ancillary device to the target site in the body. To provide such enhanced flexibility, once the tissue surrounding the access site has been sufficiently expanded or enlarged by the dilator 510 to accommodate the outer diameter OD'_{IS} of the thinner-sidewall distal section 410 of the present inventive introducer sheath, the dilator is preferably proximally withdrawn completely from the body, while maintaining the guide catheter 520 and introducer sheath 535 in position. Henceforth, the guide catheter 520 can be advanced to the target treatment site within the body, through the axial lumen defined in the present inventive introducer sheath 535, without abrading or damaging the bodily tissue at the access site shielded by the introducer sheath. Rather than using a needle, if access is realized by the interventionalist performing a "cut down" procedure using a scalpel or other surgical cutting tool, then it is contemplated and within the intended scope of the present invention that the dilator may be eliminated altogether.

The desired size of the guide catheter (e.g., 6F, 7F, 8F) comprising part of the assembled unit may be selected based on such factors as the medical procedure to be performed and ancillary devices to be used. While the present inventive introducer sheath and guide catheter remain in place to prevent injury or damage to body tissue surrounding the access site, one or more ancillary devices may be delivered through the lumen of the guide catheter over the guidewire to the target site. Once the ancillary device is properly positioned at the target site, the guidewire may be proximally withdrawn through the assembled unit and out from the body.

The present inventive vascular access system and method have been described for access of the radial artery. However, the present inventive system and method is suitable for vascular access of a target site within the vascular system (e.g., artery, vein, chamber, etc.) anywhere in the body.

Thus, while there have been shown, described, and pointed out fundamental novel features of the invention as applied to a preferred embodiment thereof, it will be understood that various omissions, substitutions, and changes in the form and details of the systems/devices illustrated, and in their operation, may be made by those skilled in the art without departing from the spirit and scope of the invention. For example, it is expressly intended that all combinations of those elements and/or steps that perform substantially the same function, in substantially the same way, to achieve the same results be within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

Every issued patent, pending patent application, publication, journal article, book or any other reference cited herein is each incorporated by reference in their entirety.

## Claims

1. A vascular access system comprising:
an assembled integral dedicated unit comprising:
an introducer sheath having a proximal end, an opposite distal end and a lumen defined axially therethrough from the proximal end to the distal end; the lumen having an inner diameter uniform along an entire length of the introducer sheath from the proximal end to the opposite distal end; the introducer sheath comprising a sidewall distal section, a sidewall proximal section and a sidewall transition section at an interface therebetween; wherein an outer diameter of the sidewall distal section is less than an outer diameter of the sidewall proximal section; and
a guide catheter having a lumen defined axially therethrough from a proximal end to an opposite distal end; the guide catheter being receivable within the lumen of the introducer sheath.

2. The system of claim 1, wherein the assembled integral dedicated unit further comprises a dilator forming a cone at a distal end; the dilator being receivable within the lumen of the guide catheter until the cone of the dilator fully extends from the distal end of the guide catheter.

3. The system of claim 1 or claim 2, wherein the sidewall transition section is an annular shoulder, a cone, or a frustum cone.

4. The system of any one of the preceding claims, wherein the outer diameter of the distal sidewall section is less than approximately 0.095" for a 6F guide catheter; less than approximately 0.110" for a 7F guide catheter, or less than approximately 0.120" for an 8F guide catheter.

5. The system of any one of the preceding claims, wherein the sidewall distal section of the introducer sheath is not self-supporting absent the guide catheter disposed in the lumen of the introducer sheath.

6. The system of any one of the preceding claims, wherein a clearance between an inner surface of the lumen of the introducer sheath and an outer surface of the guide catheter is minimized to a point of physical contact with one another.

7. A method for using a vascular access system including an assembled integral dedicated unit that comprises an introducer sheath having a proximal end, an opposite distal end and a lumen defined axially therethrough from the proximal end to the distal end; the lumen defining an inner diameter uniform along an entire length of the introducer sheath from the proximal end to the opposite distal end; the introducer sheath comprising a sidewall distal section, a sidewall proximal section and a sidewall transition section defined therebetween; wherein an outer diameter of the sidewall distal section is less than an outer diameter of the sidewall proximal section; and the integral dedicated unit further includes a guide catheter having a lumen defined axially therethrough from a proximal end to an opposite distal end; the guide catheter being receivable within the lumen of the introducer sheath; a dilator forming a cone at its distal end; wherein the vascular access system comprises the steps of:
penetrating an access site using a needle;
advancing distally through the needle an atraumatic guidewire to a target site a predetermined distance beyond the access site:
maintaining in position the guidewire while withdrawing the needle from the access site;
while the guidewire is maintained in position, assembling the dilator, guide catheter and the introducer sheath in preparation for introduction through the access site; wherein the assembling step comprises (i) inserting the cone at the distal end of the dilator into and advancing distally through the lumen of the guide catheter until the cone of the dilator fully emerges from the distal end of the guide catheter; and (ii) while radially supported by the guide catheter, sliding the introducer sheath over the guide catheter to the distal end of the guide catheter adjacent to the cone of the dilator;
back-loading the assembly including the dilator, guide catheter and introducer sheath in a proximal direction starting from a distal end of the assembly with the guidewire;
advancing the assembly together as a single unit until the dilator cone is interiorly beyond the access site so that the introducer sheath extends from interiorly of the access site to exteriorly of the access site;
withdrawing the dilator from the access site, while maintaining the assembled guide catheter and introducer sheath in position; and
advancing the guide catheter to the target site, through the axial lumen defined in the introducer sheath, without damaging the access site shielded by the introducer sheath.

8. The method of claim 7, wherein the sliding step comprises advancing the guide catheter through the lumen of the introducer sheath.

9. The method of claim 7 or claim 8, wherein the back-loading step comprises inserting a proximal end of the guidewire within a distal opening in the cone of the dilator and advancing the guidewire through the lumen defined axially therein the dilator until reaching the access site.

10. The method of any one of claims 7 to 9, wherein the outer diameter of the distal sidewall section is less than approximately 0.095" for a 6F guide catheter; less than approximately 0.110" for a 7F guide catheter, or less than approximately 0.120" for an 8F guide catheter.

11. The method of any one of claims 7 to 10, wherein the sidewall distal section of the introducer sheath is not self-supporting to pass through the access site absent structural support provided by the guide catheter disposed in the lumen of the introducer sheath.

12. The method of any one of claims 7 to 11, wherein a clearance between an inner surface of the lumen of the introducer sheath and an outer surface of the guide catheter is minimized to a point of physical contact with one another.

13. The method of any one of claims 7 to 12, wherein the vessel is a radial artery.

14. The method of any one of claims 7 to 13, wherein the sidewall transition section is an annular shoulder or a tapered cone.

15. A method for using a vascular access system including an assembled integral dedicated unit that comprises an introducer sheath having a proximal end, an opposite distal end and a lumen defined axially therethrough from the proximal end to the distal end; the lumen defining an inner diameter uniform along an entire length of the introducer sheath from the proximal end to the opposite distal end; the introducer sheath comprising a sidewall distal section, a sidewall proximal section and a sidewall transition section defined therebetween; wherein an outer diameter of the sidewall distal section is less than an outer diameter of the sidewall proximal section; and the integral dedicated unit further includes a guide catheter having a lumen defined axially therethrough from a proximal end to an opposite distal end; the guide catheter being receivable within the lumen of the introducer sheath; wherein the vascular access system comprises the steps of:
at a desired location, cutting down using a surgical cutting tool to create an access site;
advancing distally through the access site an atraumatic guidewire to a target site a predetermined distance beyond the access site:
while the guidewire is maintained in position, assembling the guide catheter and the introducer sheath in preparation for introduction through the access site; wherein the assembling step comprises, while radially supported by the guide catheter, sliding the introducer sheath over the guide catheter to the distal end of the guide catheter;
back-loading the assembly including the guide catheter and introducer sheath in a proximal direction starting from a distal end of the assembly with the guidewire;
advancing the assembly together as a single unit until interiorly beyond the access site so that the introducer sheath extends from interiorly of the access site to exteriorly of the access site; and
advancing the guide catheter to the target site, through the axial lumen defined in the introducer sheath, without damaging the access site shielded by the introducer sheath.
